# EUROPEAN PATENT APPLICATION

(11) **EP 4 400 159 A1**
(43) Date of publication of application: **17.07.2024**
(21) Application number: 24150485.1
(22) Date of filing: 05.01.2024
(51) Int. Cl.: A61N 2/02

(54) **MEDICAL APPARATUS FOR THERAPEUTIC APPLICATIONS BY MEANS OF THE USE OF MAGNETIC INDUCTION**

(30) Priority: 12.01.2023 IT 202300000285
(71) Applicant: M.F.I. S.r.l., 00163 Roma (RM) (IT)
(72) Inventor: MANTARRO, Marco, 00163 Roma (RM) (IT)
(74) Representative: Fiammenghi, Eva

(57) **Abstract**

Medical apparatus comprising at least a first control device (1) and a plurality of electromagnetic field emitting attachments, said medical apparatus being managed through a Bluetooth channel by an application running on an electronic device, the latter being a smartphone and/or a tablet, said medical apparatus being characterized in that said emitting attachments are, at least a mattress-like device (2), at least an intensive local applicator (3), at least a tip-shaped head (4), said mattress-like device (2) emitting electromagnetic fields EMF with a lower frequency component having a frequency between 0.1 and 250 Hz.

## Description

### Field of the art

The present description relates to the medical field. More in detail, the present invention concerns a peculiar apparatus comprising a plurality of attachments for use in the therapeutic treatment of subjects with specific symptoms, thanks to the delivery of electromagnetic fields, with predefined parameters, applied to extended, localized and/or point-like areas of the subject undergoing treatment.

### Known art

Rehabilitation medicine, often also known as physiatry, is that branch of medicine that deals with the prevention, diagnosis, therapy and rehabilitation of disabilities resulting from various disabling, congenital or acquired diseases. These are above all diseases that involve a limitation of activity and restrict participation in active life, through the reduction of motor, cognitive, emotional and rational functions. The areas of treatment are vast and multifaceted: the specialist addresses the disability caused by various pathological conditions and/or pain and has specific skills in the neuromuscular, osteoarticular, cognitive-relational, biomechanical, ergonomic and psychological fields. The latter combines knowledge for the management of functional cardiovascular, respiratory, uro-gynecological, metabolic and nutritional problems, not least pain management.

In this regard, it is of interest for the Applicant, for understanding the present invention described below, to recall the main aspects that fall within the field of pain therapy. In more detail, pain therapy, often also defined as analgesic therapy or pain medicine, has the aim of recognizing, evaluating and treating chronic pain in the most appropriate way. The objective is to keep the painful, chronic and often refractory stimulus at bay, in order to improve the patient's quality of life. To combat pain, there are different classes of drugs that can be used to treat pain.

The type of drug to be used can vary depending on the origin, nature and intensity of the painful stimulus that is intended to be treated. Therefore, before illustrating the categories of drugs most used in this area, it is useful to understand what chronic pain is and what causes can trigger it. However, it must be specified that in pain therapy it is also possible, as will be seen later, to also resort to non-pharmacological treatments. According to the definition provided by the International Association for the Study of Pain (IASP), pain represents "an unpleasant sensory and emotional experience associated with or similar to that of actual or potential tissue damage". Basically, it is possible to distinguish three different types of pain: acute, chronic and procedural. Chronic pain, the target of analgesic therapy, persists over time, is highly debilitating and is capable of causing not only physical, but also psychological, social and economic damage to the patients who are afflicted by it.

Very often, chronic pain is believed to be a peculiarity of neoplastic pathologies. However, tumors are not the only possible cause capable of triggering the aforesaid form of pain. In fact, the cause may not be oncological, but be for example neuropathic, or associated with degenerative pathologies of various kinds.

The choice of the type of drug to use in pain therapy depends above all on the intensity and type of pain afflicting the patient.

Below are the main classes of drugs used in analgesic therapy.

NSAIDs (non-steroidal anti-inflammatory drugs) are used in the treatment of pain when the pain is mild or moderate.

Generally, these medicines combine analgesic activity with an anti-inflammatory and antipyretic action. Their mechanism of action involves the inhibition of the cyclooxygenase enzyme, with consequent inhibition of the synthesis of prostaglandins responsible for mediating painful responses and inflammatory processes. Among the active ingredients most used in pain therapy, we point out ketoprofen, diclofenac, naproxen, and nimesulide.

Opioid analgesics, widely used in pain therapy, are particularly indicated when the painful stimulus is moderate to severe. These drugs carry out their action through the stimulation of opioid receptors present within our body. In fact, these particular receptors are located on the pain pathways and their task is precisely to mediate and transmit painful stimuli. The opioid analgesics most used in pain therapy include codeine, tramadol, buprenorphine, fentanyl, oxycodone, methadone, hydromorphone and morphine. Although they can be used in the treatment of pain of various origins and nature, the aforesaid active ingredients are considered to be the most important drugs in the treatment of chronic cancer pain.

In the context of pain therapy, it is also possible to resort to the use of cannabinoid drugs and what is known as therapeutic cannabis or marijuana for therapeutic use. The prescription of such drugs is strictly regulated and can only occur in certain and specific cases, i.e., when conventional treatments do not give satisfactory results or are no longer sufficient to control the chronic pain afflicting the patient.

The use of antidepressants is also known in the treatment of pain therapy.

Despite their main therapeutic indication in the treatment of depression, some active ingredients belonging to the classes of tricyclic antidepressants (or TCAs) and serotonin and norepinephrine reuptake inhibitors (NSRIs) have proven to be very useful in the treatment of neuropathic pain and can be used either alone or in combination with opioid analgesics. Among the most widely used TCAs in this field, we point out amitriptyline and clomipramine. Among the NSRIs, we instead point out duloxetine, an active ingredient that has proven particularly effective in the treatment of diabetic neuropathic pain.

Some types of anticonvulsant drugs have also proven to be quite effective in treating neuropathic pain. In particular, gabapentin and pregabalin are among the active ingredients most used in this area.

In fact, through the interaction with the voltage-dependent calcium channels present at the central nervous system level, these drugs are capable of reducing the release of neurotransmitters involved in the modulation and transmission of the painful stimulus, such as substance P and calcitonin gene-related peptide.

Among the local anesthetics most used in the treatment of chronic pain, we find lidocaine. More in detail, lidocaine is capable of reducing and block the transmission of painful stimuli through the inhibition of voltage-gated sodium channels present on cell membranes.

The route by which a given pain medication is administered depends on the drug itself. The most common routes include orally and by injection or infusion into the bloodstream. Depending on the type of pain that needs to be treated, it may also be necessary to resort to infiltrations (for example intra-articular, peri-articular, etc.). More invasive administration methods are added to these, which may involve: the insertion of catheters through which the drugs for pain therapy will be conveyed to the desired site; the use of infusers or elastomeric pumps that continuously and autonomously deliver the drug for the treatment of pain; patient-controlled analgesic pumps (PCA, from patient-controlled analgesia). These are devices that allow the patient to self-administer one or more pain medications carefully dosed by the doctor. Furthermore, these pumps are specifically set in such a way as to prevent the patient from being given too much medicine, foreseeing the risk of overdose. In any case, in the event of their use, the doctor and healthcare staff will provide the patient with all the necessary information.

The classic pain therapy carried out with drugs can be combined with non-pharmacological pain therapy. Naturally, the type of approach one decides to undertake varies depending on the type of chronic pain that needs to be treated. Among these different approaches, we point out radiofrequency, TENS, massage and physiotherapy. Lastly, it is now known that the psychological component also plays a certain role in the perception and possible worsening of chronic pain. For this reason, in some cases, approaches based on behavioral or cognitive psychological techniques can be useful, just as the use of relaxation techniques and distraction techniques can prove useful. The object of the present invention is to propose an innovative and peculiar device which, thanks to the emission of predefined non-ionizing radiation and the generation of specific magnetic fields, allows the therapeutic treatment of specific pathologies, primarily alleviating peripheral symptoms.

### Description of the invention

The present invention concerns a peculiar and innovative medical apparatus. The present invention also refers to said apparatus for use in the therapeutic treatment of some patients suffering from specific pathologies with equally specific symptoms. All in accordance with what is indicated in the attached claims.

More in detail, the apparatus according to the present invention has been specifically designed to perform therapeutic applications with low intensity ELF (Extra Low Frequency) electromagnetic signals in professional and non-professional environments.

Even more in detail, the main function of said apparatus is to deliver a complex ELF electromagnetic field throughout the body whose characteristics are predetermined, by means of a mattress or locally by means of a local applicator. The signals of said apparatus, at low and extra low frequency (ELF) and at very low intensity, trigger a series of chemical and physical signals and are capable of accelerating the enzymatic kinetics in that series of functions known as a whole as Reparative Morphogenesis (MR). The treatment can occur in the presence of metal implants, through plaster and with the patient dressed.

Advantageously, the apparatus according to the present invention offers greater comfort for the patient.

Advantageously, said apparatus involves fewer post-operative side effects, lower risk of surgical site infections, and less paresthesia.

Advantageously, said apparatus also leads to a reduction in healing and osteo-integration times.

Advantageously, its use is associated with greater predictability of clinical results. Advantageously, the use of the apparatus according to the present invention also promotes the regeneration of the vascular tissue; of bone tissue; of soft tissues; of nervous tissue.

Said apparatus is particularly, but not limitedly indicated in cases of edema, pain therapy, inflammation, bacterial infections.

Only for the purposes of greater descriptive completeness, it is of interest to the Applicant to point out that the use of the apparatus subject matter of the present description is not recommended on: people with pacemakers; wearers of cardiac pacemakers; pregnant women; epileptic subjects; cancer patients unless authorized by a specialist doctor; subjects with serious heart disease, unless explicitly prescribed by a specialist; oncological pathologies, unless explicitly prescribed by a specialist.

The medical apparatus according to the present invention is indicated for therapeutic and pain-relieving treatments, and is suitable for use in both hospital, outpatient and home environments.

Said apparatus is, as already mentioned, intended for the treatment of various pathologies. In particular, it is mainly, but not limitedly, intended for therapeutic, pain-relieving and regenerative use in the orthopedic, physiatric, traumatological fields and in sports medicine and regenerative medicine in general.

Furthermore, based on the tissue-specific machine codes, it is also particularly effective in the fields: dermatology, aesthetic medicine, dentistry, ophthalmology and podiatry. Such a property derives from the possibility of specifying different therapy steps, each characterized by different parameters, which can be set within a wide range of variation.

Therefore, said apparatus, depending on the set therapy parameters:
- positively modifies the dynamics of microcirculation, consequently reducing localized dystrophy phenomena;
- acts predominantly on the Ca⁺⁺ ion, excluding it from the pain transmission process;
- facilitates the course of inflammatory processes of the osteoarticular systems;
- promotes osteogenesis processes by accelerating the reconstruction process in the presence of bone fractures;
- induces an increase in blood flow in the peripheral circulatory system, accelerating the wound healing process;
- produces a muscle relaxant effect.

With reference to the indicated scope of use, which excludes critical situations, the provision of service continuity is not required.

Said apparatus can be used indifferently on people of all ages.

### Description of the figures

The medical apparatus according to the present invention will be described in detail below also with reference to the attached figures in which:

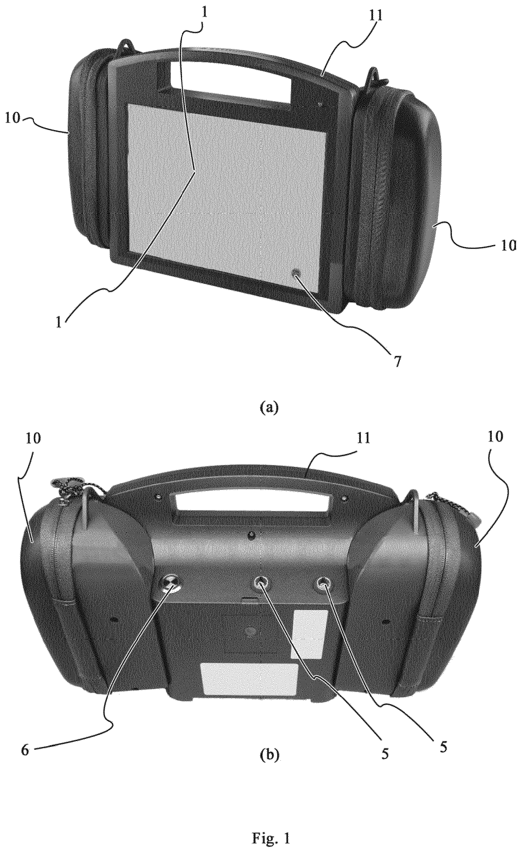

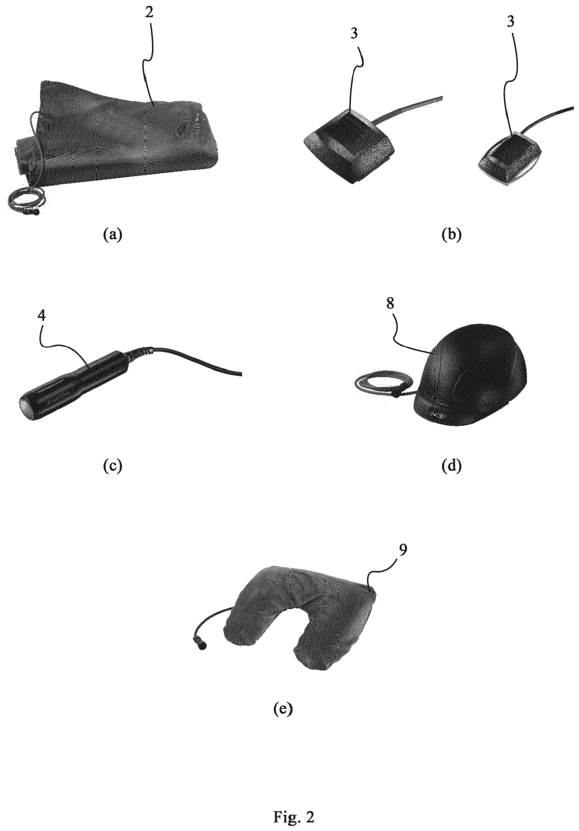

- FIGURE 1 shows a perspective view of the medical apparatus in its closed configuration like a briefcase (Fig. 1(a)). The control device 1 on which the front power switch 7 can be seen, two lateral compartments 10 for containing emitter attachments, a handle 11 are visible. Figure 2(b) also shows a perspective view, but of the rear portion, of said medical apparatus 1 in its closed configuration. The rear main switch 6 and two connectors 5 for connecting the emitter attachments contained inside said compartments 10 are visible.
- FIGURE 2 shows an exploded view of the medical apparatus according to the present invention. In more detail, the figure in question shows the types of emitter attachments comprised therein. The mattress-like device 2, (Fig. 2(a)), the intensive local applicators 3 (Fig. 2(b)), the tip-shaped head 4 (Fig. 2(c)), the multifunctional helmet 8 (Fig. 2(d)), the cervical/neck pillow 9 (Fig. 2(e)) are visible.

### Detailed description of the invention

The medical apparatus according to the present invention is represented by a set of components, each used for a specific function.

More in detail, in its preferred embodiment the apparatus according to the present invention comprises at least a first control device 1, and a plurality of attachments/emitters. More in detail, said attachments are: at least a mattress-like device 2, at least an intensive local applicator 3, at least a tip-shaped head 4.

Even more in detail, said apparatus was designed so that it could be used with the utmost simplicity, both by the specialized operator (doctor, physiotherapist, etc.) and by a common user at home. The user chooses the program from those available and, with a simple start, starts it. The attachment/emitter to be used is chosen based on the type of treatment, for example: for the treatment of a "fracture" the LIE or GPE emitters will be used, as the area to be treated is small and limited; for a problem which generally involves the whole body, the mattress-like device will be used. The choice of attachment to use does not change and does not affect the program chosen. The delivery of the program signal will always be the same, the difference lies in the fact that in a small area it will be concentrated exclusively in the affected area, while in a large area it will be distributed over the entire body.

The Applicant specifies that program is intended as: a set of steps (which can be from one to nine) forming the program itself. These steps, formed by at least four parameters (as specified below), are managed and stored exclusively by the Applicant's specialized staff and are covered by copyright. Therefore, neither the doctor nor the end user can modify them in any way.

The mattress-like device 2 of the apparatus according to the present invention emits ELF electromagnetic fields (whose lower frequency component has a frequency between 0.1 and 250Hz) of very low intensity (between 1 and 280 µT, in any case below the threshold prescribed by Directive 2013-35-EU), characterized by a notable complexity of the frequency content. In fact, in the delivered signal, in addition to the fundamental repetition frequency (between 0.1 and 250 Hz) of the selected waveform (wavelet), there is a controlled and decidedly high harmonic content, by virtue of the wavelet delivery method.

The same electromagnetic signal is delivered on at least an emitter 5 among those permitted and present, which can be from time to time, said mattress-like device 2 or local applicators 3 which multiply the intensity emitted by the mattress by a few times, concentrating it in a very localized area, sometimes point-like.

The apparatus according to the present invention, by means of said attachments, can apply a lower field intensity over a large area, as in the case of use of said mattress-like device 2, or a higher field over a very large localized area, as in the case of the local applicators 3.

The choice of the application method and thus of the emitter attachment is made based on the treatment to be performed. The intensity suitable for each case is regulated based on a large amount of clinical cases accumulated over the years.

As already mentioned, the Applicant further points out that the signals emitted by the medical apparatus according to the present invention are not ionizing radiations nor field emissions exceeding some safety limits recognized by the authorities. In fact, it should be noted that reference is being made to signal intensity values that can be interacted with every day. Therefore, it is further specified that there is no critical configuration at which the device operates.

The treatment methods, i.e., the parameters defining the electromagnetic field emitted (frequency, waveform, intensity, duration) are brought together and defined with a string of values which by historical custom is called Step. Several steps (typically nine but it can also be just one) are grouped together to achieve a certain biological effect in a sequence called a program.

The user/operator chooses to send one of the programs he can access.

Therefore, the apparatus according to the present invention is substantially, from the user's point of view, a device for delivering electromagnetic fields based on the indications in the programs dedicated to each pathology, to which the user has access. All the parameters related to the treatments are predetermined and transmitted to the apparatus by special software, created by third parties, whose certification is separate from that of the apparatus itself.

For the purposes of greater descriptive exhaustiveness, the hardware characteristics of the apparatus according to the present invention are described below.

More in detail, said apparatus comprises at least: a power circuit which accepts as inputs both a power supply, by way of non-limiting example of the "medical" type, connected to the mains, where envisaged with the batteries contained therein; a power section which simultaneously delivers the treatment on at least two channels; a section that acquires the parameters related to each treatment step (one step at a time) via Bluetooth connection, by way of non-limiting example, and without carrying out any processing, uses said parameters in order to generate the electromagnetic field.

The hardware merely carries out the execution of each step sent and is therefore not envisaged, without limitation, to have storage capacity or communication capacity, except for the Bluetooth channel.

As regards data transmission, it should be indicated that the programs are transmitted to the hardware by an application which can reside, by way of non-limiting example, on a tablet and/or a smartphone application that is the work of third parties.

It should also be noted that data transmission occurs by means of RFCOMM Bluetooth protocol and the data integrity is guaranteed:
- by the Bluetooth transmission protocol, which envisages an 8-bit HEC (Header-Error-Check) field in the header of each exchanged packet to protect against CRC errors;
- by the fact that every command received is retransmitted to the application and thus every discrepancy is detected.

The program is transferred to the apparatus from a device by means of Bluetooth and is not accessible to third parties due to its intrinsic point-to-point connection characteristic and also given the handshake at the beginning of the connection.

As regards the control logic, it is specified that: the medical apparatus according to the present invention is provided with a logic (emulated with a processor) which simply transfers the data related to each command to the relative component: for example, the received numerical value which expresses the frequency is sent to the DDS which generates the sinusoid, while the digital potentiometer is programmed with the received numerical value which expresses the amplitude and so on.

Therefore, since the operating logic is essentially reduced to the implementation of a state machine, with deterministic behavior and cannot independently determine any change to the parameters received, nor to the operational choices, it is possible to consider such logic as a component, also from the from the point of view of design development and thus the certainty of the correct behavior of the logic, together with the hardware components, is achieved through the verification of obtaining an emission of pre-established characteristics set with a pre-determined test program.

For the purposes of descriptive completeness and greater clarity of the invention, the method with which said apparatus operates for use in the therapeutic treatment of certain pathological conditions is indicated below.

In more detail, the treatment is provided in this manner:
- the appliance is turned on using the rear main switch 6 (Fig. 1(b));
- with the front power ignition switch 7 (Fig. 1(a)) the therapy delivery section is activated; then, the device waits for a Bluetooth connection;
- the application is launched from the tablet/smartphone: the Bluetooth connection with the device in question is automatically established;
- the user decides which program to deliver from the application and this is sent, one step at a time, to the hardware base;
- if envisaged by the application, the treatment can be temporarily interrupted and then resumed;
- the indication of the effective and correct operation, during the treatment, is provided by the application by means of acoustic signal at the end of each step;
- at the end of the treatment an acoustic signal is emitted (3 beeps) and the apparatus is ready to start a new cycle if necessary;
- if Bluetooth connection is lost, the apparatus switches off automatically (sends an error message);
- if the application is closed, the apparatus switches off automatically.

As already indicated, the apparatus according to the present invention comprises a plurality of attachments/emitters.

Said attachments/emitters are the mattress-like device 2, useful for treatments on the whole body, the intensive local applicators 3 designed to irradiate limited areas of the body and the tip-shaped heads 4 which allow even point-like delivery (which is useful, for example, in the treatment of acupuncture points).

The characteristic common to all the attachments is that they are essentially electrical windings in which a current of a maximum of 0.4A is made to flow, by applying a voltage of a maximum of 28 V. The conductors of said windings are insulated and furthermore the windings are enclosed in further insulating protections: this safely and completely prevents contact with the human body, even in the event of serious damage to the protections.

Again for the purpose of greater descriptive exhaustiveness, it should be noted that the application not only provides the man-machine interface and communication with the apparatus according to the present invention, but also the method for the user's possible purchase of new programs.

The application is run on commercial devices (tablets, smartphones), based on Android OS.

As already mentioned, use limits for said apparatus are recommended.

More specifically, for precautionary purposes, the use of said apparatus is prohibited with: pacemaker wearers, cardiac stimulator wearers, pregnant women, epileptic subjects, cancer patients unless authorized by a specialist doctor, subjects with heart disease, unless explicitly prescribed by a specialist.

Furthermore, use is prohibited: in the presence of anesthetic gases or pure oxygen, in the event of liquid percolating on the apparatus.

Further technical characteristics of the medical apparatus according to the present invention are listed below: power supply: from the mains, by means of power supply with 100 - 240 VAC, 50 - 60 Hz input and 28V, 2.7 A output - or with 2 batteries contained inside 12V, 1.2Ah; fuses: contained internally and cannot be replaced by the user.

The power cable for the power supply is separable, 3x0.75 mm PP2 with a length of 2.00m.

### Mattress-like device attachment 2:

The mattress-like device 2 is the part on which the patient lies to carry out the treatment on the whole body. The delivery of the electromagnetic field occurs without contact with the skin, given that the treatment occurs with the patient dressed and on the other hand the conductors generating the field are inserted in the mattress itself.

The materials with which the mattress is made are:
- fabric (warp 100% polyester titer 300 DTEX - weft 100% polyester titer 300 DTEX; fixing: coated PVC; Weight: 340 (+/-5%) gr/m2 - gray), for the external covering in contact with the patient;
- 0.5 cm expanded polyurethane sheets, for the support on which the (insulated) conductors are applied;
- the internal covering is satin lining as a sliding layer between the conductors and the external covering.

The dimensions of the mattress are by way of non-limiting example: 1800 X 700 X 20 mm.

### Intensive local applicator 3 and Tip-shaped head 4 attachments:

These attachments are made with different shapes and contain one or more ferrite cores therein capable of concentrating the electromagnetic flux on more or less large areas. They can be placed on the skin (using the certified TNT cap supplied) or on clothing and are made of POM-C or ABS material.

Said apparatus also has specific types of signals: by way of non-limiting example, said signals are of the visual, acoustic type at start-up/end of treatment/suspension/resumption.

As regards the structure of the mattress-like device 2, it is also specified that its connection with the first control device 1 occurs thanks to at least one of the connectors 5 located on the back of said first control device 1 (Fig. 1(b)) to which the mattress for the "Full Body" treatment can be connected.

This attachment is designed to generate a very uniform EM field over the entire area on which the patient lies.

In one of the preferred embodiments according to the present invention, said mattress-like device 2, depicted in Fig. 2(a), has a fabric covering (warp 100% polyester titer 300 DTEX - weft 100% polyester titer 300 DTEX; fixing: coated PVC; weight: 340 (+/-5%) g/m2 - gray color), it is soft enough to be comfortable for the patient and at the same time thin enough (approximately 2 cm) to require little space when stored folded or rolled up.

As regards the intensive local applicator 3, it is specified that: its connection with the control device 1 of the medical apparatus according to the present invention also occurs thanks to one of the connectors 5 located on the back of said control device 1 of said medical apparatus.

This attachment depicted has the purpose of generating an EMF on a limited surface (approximately 10 cm²), for localized treatments. The geometry of the delivered EMF arrives locally with a greater intensity and greater depth in the tissues, with respect to that of the mattress attachment and can be useful for localized treatments of painful symptoms. The external case of the intensive local applicator can be made indifferently, depending on the sourcing ability of the materials and the variability of the aesthetic shape (based on purely commercial reasons), in ABS, without this having an influence on performance.

The Applicant points out that the intensity of the EMF delivered by the intensive local applicator is far lower than all occupational protectionist limits.

As regards the tip-shaped head attachment 4, it is specified that its connection to the control device 1 of said medical apparatus also occurs thanks to one of the connectors located on the back of said control device 1. This attachment depicted has the purpose of generating an EMF on a specific surface (approximately 2 cm²), for extremely localized treatments or for the stimulation of acupuncture points.

The external case of the tip-shaped head can be made indifferently, depending on the sourcing ability of the materials and the variability of the aesthetic shape (based on purely commercial reasons), in ABS without this having an influence on performance. The Applicant points out that also for this attachment, the intensity of the EMF delivered by the tip-shaped head is lower than the occupational protectionist limits. Some embodiments of the present invention envisage that said medical apparatus is provided with further EMF emitting devices for use in the therapeutic treatment of specific pathological conditions.

More in detail, said embodiments also include at least a multifunctional helmet 8 and/or at least a cervical/neck pillow 9.

As regards said multifunctional helmet 8, it is specified that its connection to the control device 1 of said medical apparatus also occurs in this case thanks to at least one of the connectors located on the back of said control device 1. Said multifunctional helmet 8 has the purpose of generating a low intensity EMF on the internal surface.

The helmet is a normal training cap adaptable to all people without any distinction.

It is made of plastic material with the internal lining in soft fabric to make the treatment comfortable.

In the event of treatment, the person must wear a disposable cap, also made of nonwoven fabric, for hygienic purposes without this having an influence on performance. The intensity of the EMF delivered inside the multifunctional helmet is lower than the occupational protection limits.

As regards the cervical/neck pillow 9, it is specified that, also in this case, its connection to the control device 1 of the medical apparatus according to the present invention occurs thanks to at least one of the connectors 5 located on the back of said control device 1.

This attachment has the purpose of generating a weak intensity EMF on the internal surface.

It can be adapted to all people without any distinction.

It is made with cotton fabric material with the interior in soft fabric to make the treatment comfortable.

In the event of treatment, the person must wear a disposable cover, also made of nonwoven fabric, for hygienic purposes without this having an influence on performance. The intensity of the EMF delivered in the treatment area is lower than the occupational protectionist limits.

Only for the purposes of greater descriptive exhaustiveness, it is specified that as regards the electrical diagrams, said medical apparatus comprises: a main board, called motherboard, and a board related to power management and battery charging. The circuits are easily interpretable by any person skilled in the art and therefore their characteristics are omitted from the present description, taking it for granted that their presence and functionality are implied by the person skilled in the art.

As indicated several times throughout the present description and in accordance with what is specified in the attached claims, the medical apparatus according to the present invention can be used in the treatment of various pathologies that reveal inflammatory symptoms causing pain; therefore, said apparatus proves to be a valid tool primarily for use in the therapeutic treatment of pain. Furthermore, said apparatus proves to be particularly useful in the treatment of rheumatological diseases. Said diseases can then be divided into four large categories depending on the area of the body mainly affected, the symptoms and the cause of onset. These are: degenerative diseases, inflammatory diseases; systemic autoimmune diseases caused by an abnormal response of the immune system that generates persistent inflammation at the level of multiple organs; metabolic diseases, linked to errors in the turnover of certain substances in the body. Among the most common rheumatological diseases are: osteoarthritis (a degenerative disease in which the cartilage that covers the bones at the joint level thins and fragments. This disease involves pain and motor difficulties, the joints most affected are: spine, hands, feet, hips and knees); rheumatoid arthritis (a form of chronic autoimmune inflammatory disease involving the joints of the hands, feet, wrists, ankles, knees, hips, elbows and shoulders whose painful symptoms make simple daily actions difficult if not impossible; Spondylo-entheso-arthritis (a group of chronic diseases characterized by inflammatory back pain and enteritis, i.e., inflammation at the junction between tendons, ligaments and bone); Ankylosing spondylitis (a form of arthritis leading to a progressive stiffening of the spine and with forced flexion of the neck and inability to flex the spine. It is generally more frequent in men); Extra articular rheumatism (mainly involving tendons, ligaments, synovial bags and muscles. Gout: metabolic disease caused by excessive production or poor elimination of uric acid (through urine). Urate crystals are deposited in the joints which cause inflammation, swelling, redness and intense pain, especially in the big toe. Also the hands, ankles, wrists and knees can be affected; Osteoporosis (a very frequent pathology in the elderly, characterized by a reduction in bone mineral density associated with bone deterioration resulting in skeletal fragility. This translates into a greater risk of fractures").

It should also be noted that the apparatus according to the present invention is also used in the treatment of chronic pain in general, the causes of which can also be traced back to states of depression in the patient.

Furthermore, following the type of signals used and their delivery methods, the present invention is particularly useful in the field of regenerative medicine.

## Claims

1. Medical apparatus comprising at least a first control device (1) and a plurality of electromagnetic field emitting attachments, said medical apparatus being managed through a Bluetooth channel by an application running on an electronic device, like a smartphone and/or tablet, said medical apparatus being **characterized in that** said emitting attachments include at least a mattress-like device (2), at least an intensive local applicator (3), and at least a tip-shaped head (4), wherein said mattress-like device (2) emits electromagnetic fields EMF whose lowest frequency component has a frequency between 0.1 and 250 Hz and said EMF have an intensity between 1 µT and 280 µT, wherein said first control device (1) is provided with at least two connectors (5) which are apt to supply each time at least said mattress-like device (2) or the intensive local applicators (3) or the tip-shaped heads (4), wherein said tip-shaped heads (4) and said intensive local applicators (3) instead amplify the intensity emitted by the mattress-like device by focusing/concentrating this intensity in restricted/local areas, that may be either of larger size or point-like; wherein said electromagnetic field emitting attachments are selected according to the kind of treatment to be carried out, and said electromagnetic field emitting attachments radiate non-ionizing radiation; wherein said apparatus further comprises at least a power circuit which accepts as inputs a power supply or a battery case, if any; a power section which provides on at least two channels a specific predetermined treatment; a section adapted for acquiring through the Bluetooth connection parameters related to each step of the treatment and for using said parameters in order to generate an electromagnetic field, wherein said apparatus generates magnetic fields obtained from signals whose waveform is selectable from pulsed waveforms, triangular waveforms, sinusoidal waveforms, rectangular waveforms, and comprising multiple harmonics and with application/delivery times between one and four minutes; wherein said magnetic field is applied by taking into account the physiological functions/responses one wants to activate depending on the biologic plan/program for healing to be realized/developed, which essentially aims at re-balancing the cell system.

2. Medical apparatus according to the preceding claim, further comprising a rear main switch (6) for turning the apparatus on, and a front power switch (7) for the activation of the therapy section.

3. Medical apparatus according to anyone of the preceding claims, wherein the mattress-like device (2) comprises, among the materials from which it is realized, polyester, expanded polyurethane panels with insulated conductors applied thereon, and a smooth internal lining.

4. Medical apparatus according to the preceding claim, wherein the expanded polyurethane panels of said mattress-like device (2) have a thickness of 0.5 cm and the dimensions of the mattress-like device (2) correspond to 1800 x 700 x 200 cm.

5. Medical apparatus according to anyone of the preceding claims, wherein the electromagnetic field emitting attachments, like the intensive applicator (3) and the tip-shaped head (4), include in their interior at least a ferrite core apt to concentrate the electromagnetic flux on more or less extensive areas.

6. Medical apparatus according to the preceding claim, wherein both the intensive local applicator (3) and the tip-shaped head (4) are provided with an external casing made of ABS.

7. Medical apparatus according to anyone of the preceding claims, wherein an additional electromagnetic field emitting attachment is provided, in the form of a multifunctional helmet (8), adapted for applying an EMF lower than the exposure limit values for workers, wherein said multifunctional helmet is made of plastics and has an inner lining made of soft fabric.

8. Medical apparatus according to anyone of the preceding claims, wherein as a further electromagnetic field emitting attachment, a cervical/neck pillow (9) is provided that is apt to apply EMFs less than the required safe exposure limits, said pillow being made of soft fabric.

9. Medical apparatus according to anyone of the preceding claims, wherein among the electromagnetic field emitting attachments, one or more pairs of glasses are also provided.

10. Medical apparatus according to anyone of the preceding claims, having a closed configuration/shape or an open configuration/shape, wherein as concerns the closed configuration/shape, this has the form of a small suitcase equipped with a handle (11) and having at least two lateral compartments (10) for containing the electromagnetic field emitting attachments.

11. Medical apparatus according to anyone of the preceding claims, for use in a method of therapeutic treatment of pathologies associated with pain.

12. Medical apparatus for use in accordance with the preceding claim, wherein the pathology is arthrosis; rheumatoid arthritis; spondylo-entheso-arthritis; ankylosing spondylitis; extra articular rheumatism; gout; osteoporosis, depression.

13. Medical apparatus for use according to claim 11 in a method of treatment of edemas, phlogosis, bacterial infections.

14. Medical apparatus for use according to claim 11 in a method of treatment of regenerative medicine in general.
